# FASCICULE DE BREVET EUROPEEN

(11) **EP 0 919 551 B1**
(45) Date de publication et mention de la délivrance du brevet: **26.02.2003**
(21) Numéro de dépôt: 98204008.1
(22) Date de dépôt: 24.11.1998
(51) Int. Cl.: C07D 303/08, C07D 301/12, C07D 301/32

(54) **Procédé pour la fabrication de épichlorhydrine très pure**
Verfahren zur Herstellung von Reinst-Epichlorhydrin
Process for the manufacture of very pure epichlorohydrin

(30) Priorité: 27.11.1997 BE 9700961
(43) Date de publication de la demande: 02.06.1999
(73) Titulaire: SOLVAY (Société Anonyme), 1050 Bruxelles (BE)
(72) Inventeur: Strebelle, Michel, 1150 Bruxelles (BE); Gilbeau, Patrick, 7090 Braine-le-Comte (BE); Catinat, Jean-Pierre, 7131 Waudrez (BE)
(74) Mandataire: Vande Gucht, Anne

(56) Documents cités:
- EP-A- 0 100 119
- EP-A- 0 230 949
- EP-A- 0 336 077
- EP-A- 0 368 656
- EP-A- 0 568 336

## Description

La présente invention se rapporte à un procédé de fabrication d'un produit à base d'épichlorhydrine par réaction entre le chlorure d'allyle et un composé peroxydé dans un milieu liquide contenant un diluant, et plus particulièrement à un procédé amélioré de fabrication de ce produit appauvri en impuretés chlorées.

Il est bien connu de préparer l'épichlorhydrine par déshydrochloration, à l'aide d'un composé basique, d'une solution aqueuse de dichloropropanols, laquelle est obtenue en faisant réagir, dans une zone réactionnelle appropriée, du chlorure d'allyle, de l'eau et du chlore, comme décrit par exemple dans la demande de brevet EP-A1-561441.

Au cours de ce procédé connu se forment généralement des sous-produits non désirés, à savoir des produits organiques chlorés. Ces produits étant difficiles à éliminer, on peut en retrouver une partie dans l'épichlorhydrine. De plus, ces sous-produits posent des problèmes de rejet car ils contribuent à la demande chimique en oxygène et, le cas échéant, à la présence de composés halogénés indésirables.

Il est également connu de préparer de l'épichlorhydrine en utilisant du chlorure d'allyle et du peroxyde d'hydrogène en présence d'eau, d'un diluant et d'un catalyseur, comme dans la demande de brevet EP 568336.

L'invention vise à remédier aux inconvénients précités en fournissant un produit plus pur contenant moins d'impuretés et surtout moins d'impuretés chlorées ainsi qu'un procédé simple de préparation d'épichlorhydrine qui génère moins de sous-produits et surtout moins de sous-produits chlorés et qui présente une haute sélectivité.

L'invention concerne dès lors un procédé de fabrication d'un produit à base d'épichlorhydrine, selon lequel
(a) on fait réagir du chlorure d'allyle avec un composé peroxydé en présence d'eau, d'un catalyseur et de méthanol comme diluant dans au moins un réacteur, et
(b) on soumet le mélange réactionnel sortant de l'étape (a) à un traitement de séparation de l'épichlorhydrine par extraction au moyen d'un solvant d'extraction et on recueille d'une part, un extrait contenant au moins une partie du solvant d'extraction, au moins 10 % de l'épichlorhydrine produite, éventuellement un excès de chlorure d'allyle et des traces de méthanol, et, d'autre part, un raffinat contenant au moins une partie du diluant, au moins une partie de l'eau, des traces d'épichlorhydrine et des sous-produits
(c) on soumet l'extrait à un traitement de distillation afin de séparer une première fraction contenant l'épichlorhydrine produite, éventuellement l'excès de chlorure d'allyle et des traces de méthanol d'une deuxième fraction contenant le solvant d'extraction que l'on recycle dans l'étape d'extraction (b).

Le procédé selon l'invention permet d'obtenir un produit à base d'épichlorhydrine contenant au moins 99,9 % en poids d'épichlorhydrine. Ce produit peut contenir une quantité totale d'impuretés chlorées inférieure ou égale à 150 ppm en poids, en particulier inférieure ou égale à 100 ppm. Ce produit contient avantageusement une quantité de méthylglycidyléther inférieure ou égale à 250 ppm en poids, en particulier inférieure ou égale à 200 ppm. Il contient de préférence une quantité de 2-méthoxypropan-1-ol inférieure ou égale à 100 ppm en poids, en particulier inférieure ou égale à 80 ppm.

Par impuretés et sous-produits, on entend désigner des produits qui sont formés par réaction entre l'épichlorhydrine et de l'eau ou éventuellement le diluant et par réaction entre le chlorure d'allyle et le diluant. Par exemple, l'épichlorhydrine et l'eau ou le méthanol utilisé comme diluant peuvent former, dans les conditions habituelles d'époxydation, des quantités notables de 1-chloro-3-méthoxypropan-2-ol, de 1-chloro-2-méthoxypropan-3-ol, de 1,3-dichloropropan-2-ol, de 2,3-dichloropropanol et de 1-chloro-2,3-dihydroxypropane. Par ailleurs, la réaction entre le chlorure d'allyle et le méthanol utilisé comme diluant peut donner du méthylallyléther qui, dans les conditions d'époxydation avec un composé peroxydé tel que le peroxyde d'hydrogène, peut donner du méthylglycidyléther. On peut également retrouver, parmi les impuretés et sous-produits, des produits formés par réaction entre l'eau ou le diluant et des produits secondaires éventuellement présents dans le chlorure d'allyle de départ. C'est le cas par exemple du 2-méthoxypropan-1-ol.

Les catalyseurs qui peuvent être utilisés dans l'étape (a) du procédé selon l'invention sont de préférence des catalyseurs de type silicalite au titane. Ce sont des matériaux synthétiques cristallins de structure analogue à celle des zéolithes, comprenant des oxydes de silicium et de titane et caractérisés par une bande d'absorption infrarouge à environ 950-960 cm⁻¹. Leur formule générale est typiquement :

xTiO₂.(1-x)SiO₂

dans laquelle x est compris entre 0,0001 et 0,5, de préférence entre 0,001 et 0,05.

Des matériaux de ce type, connus sous le nom de TS-1, présentent une structure zéolithique cristalline microporeuse analogue à celle de la zéolithe ZSM-5. Les propriétés et les principales applications de ces composés sont connues (B. Notari, Structure-Activity and Selectivity Relationship in Heterogeneous Catalysis, R.K. Grasselli and A.W. Sleight Editors, Elsevier, 1991, p. 243-56). Leur synthèse a été étudiée notamment par A. Van der Poel et J. Van Hooff (Applied Catalysis A, 1992, Vol. 92, p. 93-111). D'autres matériaux de ce type ont une structure analogue à celle de la zéolithe bêta ou de la zéolithe ZSM-11.

Les catalyseurs sont en général mis en oeuvre en quantités supérieures à 2, le plus souvent supérieures à 5 et de préférence supérieures à 10 g par kilo de mélange réactionnel. Ces quantités ne dépassent en général pas 200 et de préférence pas 100 g par kilo de mélange réactionnel.

Le composé peroxydé qui peut être utilisé dans l'étape (a) du procédé selon l'invention peut être choisi parmi le peroxyde d'hydrogène et tout composé peroxydé contenant de l'oxygène actif et capable d'effectuer une époxydation. On peut citer à titre d'exemples les composés peroxydés obtenus par oxydation de composés organiques tels que l'éthylbenzène, l'isobutane et l'isopropanol. Le peroxyde d'hydrogène est préféré.

Le composé peroxydé peut être mis en oeuvre sous la forme d'une solution aqueuse ou sous la forme d'une solution organique. Pour des raisons économiques, on le met en général en oeuvre sous la forme d'une solution aqueuse.

Lorsque le composé peroxydé est le peroxyde d'hydrogène, des solutions contenant au moins 20 % et, de préférence, au moins 30 % en poids de peroxyde d'hydrogène conviennent bien. Les solutions mises en oeuvre peuvent contenir jusqu'à 85 % en poids de peroxyde d'hydrogène. De préférence, on utilise des solutions contenant moins de 40 % en poids de peroxyde d'hydrogène. De manière particulièrement préférée, on utilise des solutions contenant environ 35 % en poids de peroxyde d'hydrogène.

On peut opérer dans l'étape (a) avec un rapport molaire chlorure d'allyle sur composé peroxydé qui peut varier dans de larges limites. Le rapport molaire est généralement d'au moins 0,5, en particulier d'au moins 1. Le rapport molaire est habituellement inférieur ou égal à 10, en particulier à 4.

La faible miscibilité des réactifs, le chlorure d'allyle et la solution aqueuse de composé peroxydé, rend nécessaire l'utilisation d'un diluant commun dans l'étape (a). Le diluant utilisé dans l'étape (a) du procédé selon l'invention est le méthanol.

Le diluant peut être mis en oeuvre en quantités variables. En général, le mélange réactionnel contient au moins 30 % en poids de diluant, le plus souvent au moins 50 % en poids. Habituellement, il en contient au plus 90 % en poids.
De préférence, il n'en contient pas plus de 75 %.

La température et la pression auxquelles on opère l'étape (a) peuvent varier dans de très larges limites. Elles sont choisies de manière à ne pas dépasser la température de décomposition du mélange réactionnel.

La température de l'étape (a) est habituellement inférieure à 150 °C et le plus souvent comprise entre 0 et 120 °C. De bons résultats ont été obtenus à des températures comprises entre 20 et 80 °C.

La pression dans l'étape (a) peut être inférieure, égale ou supérieure à la pression atmosphérique. La pression est en général inférieure à 5 bar. De bons résultats ont été obtenus en utilisant des pressions de 0,05 à 3 bar.

La durée de l'étape (a) dépend du catalyseur, du composé peroxydé, du diluant et des quantités mises en oeuvre de chacun des constituants. Elle est choisie de manière à obtenir un taux de conversion du composé peroxydé très élevé à complet. Elle peut aller de 1 minute à 50 heures. De bons résultats ont été obtenus avec une durée de réaction de 5 minutes à 2 heures.

L'étape (a) peut être mise en oeuvre dans un réacteur unique ou dans une série de réacteurs en parallèle ou en série. Pour réaliser l'étape (a) du procédé selon l'invention, on peut utiliser n'importe quel appareillage convenant pour les mélanges réactionnels liquides. On peut ainsi, par exemple, utiliser un ou plusieurs réacteur(s) à lit fixe, à lit transporté, à lit agité ou à lit fluidisé. De préférence, on utilisera un ou plusieurs réacteur(s) en série dans le(s)quel(s) le catalyseur est maintenu en suspension par fluidisation. On utilise de manière particulièrement préférée au moins trois réacteurs en série.

La réaction est exothermique. A chaque réacteur, la chaleur de réaction peut être éliminée par circulation du mélange réactionnel sur un refroidisseur.

Le catalyseur utilisé dans l'étape (a) peut être séparé du mélange réactionnel par une méthode appropriée comme la filtration. Il peut être économiquement avantageux de réutiliser le catalyseur récupéré dans des réactions d'époxydation ultérieures. Pour pouvoir réutiliser le catalyseur, il est avantageux de le régénérer par une technique telle que la calcination, le traitement à l'aide d'un solvant ou la mise en contact avec une solution liquide comprenant au moins un agent oxydant tel que le peroxyde d'hydrogène, l'ozone ou un composé peroxydé organique comme l'acide performique ou l'acide peracétique.

Le mélange réactionnel sortant de l'étape (a) contient généralement au moins 1 % en poids d'épichlorhydrine, le plus souvent au moins 5 % en poids. Habituellement, il en contient au plus 50 % en poids. De préférence, il n'en contient pas plus de 20 %.

Typiquement, le mélange réactionnel sortant de l'étape (a) contient de 5 à 25 % d'eau.

La teneur en chlorure d'allyle non converti dans le mélange réactionnel sortant de l'étape (a) est généralement de 5 à 20 % en poids.

Le traitement de séparation (b) de l'épichlorhydrine est effectué par extraction au moyen d'un solvant d'extraction.

Le solvant d'extraction utilisable dans l'étape (b) peut contenir un ou plusieurs composés. Avantageusement, on utilise un solvant d'extraction qui dissout bien l'épichlorhydrine et dans lequel le diluant est très peu soluble. De préférence, on utilise un solvant d'extraction qui, en outre, dissout bien le chlorure d'allyle de départ.

Des composés qui peuvent être utilisés comme solvant d'extraction dans le traitement de séparation (b) de l'épichlorhydrine sont les hydrocarbures saturés éventuellement halogénés comportant de 3 à 20 atomes de carbone, linéaires ou ramifiés, aliphatiques ou cycliques. On peut citer à titre d'exemples notamment le n-décane, le n-tridécane, le 1,2,3-trichloropropane et la décaline (décahydronaphtalène).

Le solvant d'extraction de l'étape (b) peut également être choisi parmi les hydrocarbures insaturés éventuellement halogénés comportant de 3 à 20 atomes de carbone. On peut citer à titre d'exemple le chlorure d'allyle.

D'autres composés utilisables comme solvant d'extraction dans l'étape (b) sont les hydrocarbures aromatiques contenant éventuellement des substituants alkylés, halogénés et/ou azotés, comportant de 6 à 12 atomes de carbone. On peut citer à titre d'exemples les o-, m- et p-xylènes, le 1,3,5-triméthylbenzène (mésitylène), les o-, m- et p-dichlorobenzènes, les o-, m- et p-chlorotoluènes et le nitrobenzène.

Il peut être avantageux d'utiliser dans l'étape (b) un mélange d'au moins deux solvants différents. Il peut par exemple s'agir de mélanges d'un hydrocarbure aromatique tel que décrit plus haut avec un hydrocarbure aliphatique tel que décrit plus haut. D'autres mélanges qui peuvent convenir sont les mélanges d'hydrocarbures aliphatiques et les mélanges d'hydrocarbures aromatiques.

Des solvants d'extraction particulièrement performants pour l'étape (b) contiennent au moins un composé choisi parmi l'o-dichlorobenzène, le m-dichlorobenzène, le 1,3,5-triméthylbenzène, la décaline, l'o-chlorotoluène, le 1,2,3-trichloropropane, le chlorure d'allyle, le nitrobenzène, le n-décane et leurs mélanges. Des solvants d'extraction contenant l'o-dichlorobenzène sont tout particulièrement préférés.

On recueille, après extraction dans l'étape (b), d'une part, un extrait contenant au moins une partie du solvant d'extraction, au moins 10 % de l'épichlorhydrine produite, éventuellement un excès de chlorure d'allyle et des traces du diluant, et, d'autre part, un raffinat contenant au moins une partie du diluant, au moins une partie de l'eau, des traces d'épichlorhydrine et des sous-produits. On peut ensuite traiter séparément ledit extrait et ledit raffinat.

On soumet l'extrait à un traitement de distillation (c) afin de séparer une première fraction contenant l'épichlorhydrine produite, éventuellement l'excès de chlorure d'allyle et des traces de diluant d'une deuxième fraction contenant le solvant d'extraction que l'on recycle dans l'étape d'extraction (b).

Ensuite, on peut soumettre la première fraction obtenue dans l'étape de distillation (c) à un traitement de distillation (d) afin d'éliminer l'éventuel excès de chlorure d'allyle et les traces de diluant que l'on recycle dans l'étape d'époxydation (a). Ensuite, on recueille un produit à base d'épichlorhydrine qui peut être séché dans une colonne azéotropique.

De même, on peut soumettre le raffinat obtenu dans l'étape d'extraction (b) à une distillation (e), afin de séparer un premier fluide contenant l'eau et des sous-produits d'un deuxième fluide contenant le diluant et des traces d'épichlorhydrine que l'on recycle dans l'étape d'époxydation (a).

On peut ensuite soumettre le premier fluide à un traitement d'épuration (f) afin d'éliminer les sous-produits. Le traitement d'épuration (f) peut être réalisé par distillation en présence d'un composé organique ou par extraction au moyen d'un liquide d'extraction.

Lorsque le traitement d'épuration (f) du premier fluide contenant l'eau et des sous-produits est réalisé par distillation en présence d'un composé organique, le procédé consiste à ajouter au premier fluide un composé organique et à soumettre le mélange contenant le premier fluide et le composé organique à un traitement de distillation.

Ce procédé de distillation (f) peut être effectué selon les méthodes classiques de distillation azéotropique.

Après la distillation (f), on peut recueillir, d'une part, en tête de distillation, une première phase liquide contenant le composé organique et une deuxième phase liquide contenant de l'eau épurée en sous-produits et d'éventuelles traces de composé organique, et d'autre part, en pied de distillation, un mélange de composé organique et de sous-produits. Les deux phases liquides distinctes recueillies en tête de distillation peuvent être séparées selon les méthodes classiques de séparation telles que la décantation. Ainsi, on récupère, d'une part, la première phase liquide contenant le composé organique que l'on peut recycler à la distillation (f), tel quel ou après l'avoir soumis à un traitement d'épuration. D'autre part, on récupère la deuxième phase liquide, contenant l'eau épurée en sous-produits et d'éventuelles traces de composé organique. Ces traces de composé organique peuvent, éventuellement, être récupérées par stripping pour être recyclées à la distillation (f). Ensuite, on peut également soumettre le pied de distillation, qui contient un mélange de composé organique et de sous-produits, à une évaporation, éventuellement sous vide, afin de récupérer le composé organique à l'état épuré et de le recycler à la distillation (f).

Le composé organique utilisable dans l'étape de distillation (f) peut contenir un ou plusieurs composés. Généralement, on utilise un composé organique qui présente une miscibilité très faible avec l'eau.

Des composés organiques qui peuvent être utilisés pour la distillation (f) du premier fluide contenant l'eau et des sous-produits sont les dérivés organiques aliphatiques ou aromatiques pouvant inclure des atomes tels que l'oxygène et/ou un halogène, ainsi que leurs mélanges. On peut citer à titre d'exemples les hydrocarbures aromatiques alkylés portant un ou plusieurs groupes alkyles contenant de 1 à 4 atomes de carbone comme le toluène, le xylène, le 1,3,5-triméthylbenzène, l'éthylbenzène et le butylbenzène. Le xylène est particulièrement préféré. Par xylène, on entend désigner aussi bien les o-, m- et p-xylènes que leurs mélanges. On peut également citer les hydrocarbures aliphatiques saturés contenant de 5 à 12 atomes de carbone comme le pentane, l'hexane, l'octane et le décane, ainsi que des hydrocarbures aliphatiques cycliques comme la décaline.

Lorsque le traitement d'épuration (f) du premier fluide contenant l'eau et des sous-produits est réalisé par extraction au moyen d'un liquide d'extraction, on utilise généralement un liquide d'extraction qui présente une miscibilité très faible avec l'eau. Le liquide d'extraction peut contenir un ou plusieurs composés.

Des composés qui peuvent être utilisés comme liquide d'extraction dans le traitement (f) du premier fluide contenant l'eau et des sous-produits sont les dérivés organiques aliphatiques ou aromatiques pouvant inclure des atomes de soufre, de phosphore, d'azote, d'oxygène et/ou un halogène. On peut citer à titre d'exemples les trialkylphosphines-oxyde et le 1,2-dichloropropane. Ce dernier s'avère tout particulièrement intéressant car il est formé comme sous-produit dans le procédé de fabrication selon l'invention. Les trialkylphosphines-oxyde dont chacun des groupes alkyle contient de 2 à 20 atomes de carbone, en particulier de 4 à 10 atomes de carbone, conviennent bien. La trihexylphosphine-oxyde, la trioctylphosphine-oxyde, la (octyl, dihexyl)phosphine-oxyde, la (hexyl, dioctyl)phosphine-oxyde et leurs mélanges sont particulièrement préférés.

Le procédé selon l'invention peut être mis en oeuvre en continu, en semi-continu ou en discontinu.

Le procédé selon l'invention présente l'avantage de conduire à un volume d'effluent aqueux réduit (de 1 à 1,5 m³ d'effluent par tonne d'épichlorhydrine produite). Par effluent aqueux, on entend désigner le premier fluide contenant l'eau et des sous-produits issu de l'étape de distillation (e).

Il présente aussi l'avantage d'aboutir à une sélectivité élevée de la réaction. Lorsque le procédé est mis en oeuvre en continu, des sélectivités supérieures ou égales à 98 % peuvent être atteintes. Lorsque le procédé est mis en oeuvre en discontinu, des sélectivités supérieures ou égales à 99 % peuvent être atteintes.

Ce procédé présente également l'avantage de ne pas donner lieu lors de l'étape (a) d'époxydation à une décomposition significative du peroxyde d'hydrogène en oxygène.

L'exemple qui suit est destiné à illustrer la présente invention sans toutefois en limiter la portée.

### Exemple

L'installation comporte deux réacteurs tubulaires verticaux, thermostatisés, disposés en cascade. Un lit fixe de catalyseur est placé dans chaque réacteur. Chaque réacteur est affublé d'une boucle et d'une pompe permettant la recirculation du liquide sur le catalyseur. Une homogénéisation de la température est assurée par un serpentin réfrigérant dans chaque réacteur. Le volume du lit catalytique dans chaque réacteur est de 125 ml pour un volume total (réacteur + boucle de recirculation) de 300 ml.

On a disposé dans chaque réacteur 7,4 g de catalyseur TS-1. Le réacteur est alimenté en continu à un débit de 375 ml/h (337 g/h) par une solution de chlorure d'allyle et de peroxyde d'hydrogène dans du méthanol (chlorure d'allyle/H₂O₂ = 2 mol/mol ; concentration en H₂O₂ dans la solution introduite = 1,34 mol/kg) à la température de 10 °C. Le temps de séjour du mélange de réactifs sur le catalyseur est de 20 minutes. Le mélange de réactifs (chlorure d'allyle, peroxyde d'hydrogène et méthanol) est préparé juste avant son introduction à débit constant en tête du premier réacteur.

La vitesse linéaire de passage de la solution en recirculation dans chaque réacteur a été réglée à 0,94 ml/min et le débit de recirculation est de l'ordre de 30 l/h.

La durée des essais a été fixée sur base d'une décroissance de 25 % de l'activité initiale du catalyseur dans le premier réacteur après une mise en régime d'une heure.

Dans ces conditions et après une durée de fonctionnement de 6 heures, on récupère un mélange réactionnel contenant 78 g d'épichlorhydrine. La sélectivité en épichlorhydrine (c'est-à-dire le rapport molaire entre la quantité d'épichlorhydrine produite et la somme des quantités de produits formés) est de 99,1 % sur base du chlorure d'allyle consommé.

Ce mélange réactionnel est soumis à un traitement d'extraction par de l'o-dichlorobenzène (2,5 kg par kg de mélange réactionnel) dans une colonne à plateaux. Le dispositif permet d'extraire 99 % de l'épichlorhydrine produite. Une première étape de distillation de l'extrait permet de récupérer une première fraction, en tête de distillation, contenant l'épichlorhydrine produite, le chlorure d'allyle non converti et des traces de méthanol, et une deuxième fraction, en pied de distillation, contenant l'o-dichlorobenzène qui est recyclé à l'étape d'extraction. Dans une deuxième étape de distillation, on sépare, en tête de distillation, le chlorure d'allyle et des traces de méthanol avant de recueillir le produit à base d'épichlorhydrine et de le sécher dans une colonne azéotropique. Le produit à base d'épichlorhydrine est ensuite récupéré en phase gazeuse. Il ne contient comme impuretés chlorées que 10 ppm de 1-chloro-3-méthoxypropan-2-ol et 35 ppm de 1,3-dichloropropan-2-ol et comme impuretés non chlorées que 100 ppm de méthylglycidyléther et 40 ppm de 2-méthoxypropan-1-ol.

## Revendications

1. Procédé de fabrication d'un produit à base d'épichlorhydrine, selon lequel
(a) on fait réagir du chlorure d'allyle avec un composé peroxydé en présence d'eau, d'un catalyseur et de méthanol comme diluant dans au moins un réacteur, et
(b) on soumet le mélange réactionnel sortant de l'étape (a) à un traitement de séparation de l'épichlorhydrine par extraction au moyen d'un solvant d'extraction et on recueille d'une part, un extrait contenant au moins une partie du solvant d'extraction, au moins 10 % de l'épichlorhydrine produite, éventuellement un excès de chlorure d'allyle et des traces de méthanol, et, d'autre part, un raffinat contenant au moins une partie du diluant, au moins une partie de l'eau, des traces d'épichlorhydrine et des sous-produits
(c) on soumet l'extrait à un traitement de distillation afin de séparer une première fraction contenant l'épichlorhydrine produite, éventuellement l'excès de chlorure d'allyle et des traces de méthanol d'une deuxième fraction contenant le solvant d'extraction que l'on recycle dans l'étape d'extraction (b).

2. Procédé selon la revendication 1, dans lequel on soumet la première fraction obtenue dans l'étape de distillation (c) à un traitement de distillation (d) afin d'éliminer l'éventuel excès de chlorure d'allyle et les traces de méthanol que l'on recycle dans l'étape d'époxydation (a), et on recueille un produit à base d'épichlorhydrine qui est ensuite séché dans une colonne azéotropique.

3. Procédé selon la revendication 1 ou 2, dans lequel on soumet le raffinat obtenu dans l'étape d'extraction (b) à une distillation (e), afin de séparer un premier fluide contenant l'eau et des sous-produits d'un deuxième fluide contenant le méthanol et des traces d'épichlorhydrine que l'on recycle dans l'étape d'époxydation (a).

4. Procédé selon la revendication 3, dans lequel on soumet le premier fluide à un traitement d'épuration (f) afin d'éliminer les sous-produits et dans lequel le traitement d'épuration est réalisé par distillation en présence d'un composé organique ou par extraction au moyen d'un liquide d'extraction.

5. Procédé selon l'une quelconque des revendications 1 à 4, dans lequel le composé peroxydé est le peroxyde d'hydrogène.

6. Procédé selon l'une quelconque des revendications 1 à 5, dans lequel le produit à base d'épichlorhydrine contient au moins 99,9 % en poids d'épichlorhydrine.

7. Procédé selon l'une quelconque des revendications 1 à 6, dans lequel le produit à base d'épichlorhydrine contient des impuretés chlorées en une quantité totale inférieure ou égale à 150 ppm en poids.

8. Procédé selon l'une quelconque des revendications 1 à 7, dans lequel le produit à base d'épichlorhydrine contient du méthylglycidyléther en une quantité inférieure ou égale à 250 ppm en poids.

9. Procédé selon l'une quelconque des revendications 1 à 8, dans lequel le produit à base d'épichlorhydrine contient du 2-méthoxypropan-1-ol en une quantité inférieure ou égale à 100 ppm en poids.

10. Procédé selon l'une quelconque des revendications 7 à 9, dans lequel la quantité totale d'impuretés chlorées est inférieure ou égale à 100 ppm, la quantité de méthylglycidyléther est inférieure ou égale à 200 ppm, et la quantité de 2-méthoxypropan-1-ol est inférieure ou égale à 80 ppm.

## Claims

1. Process for manufacturing an epichlorohydrin-based product, according to which:
(a) allyl chloride is reacted with a peroxide compound in the presence of water, a catalyst and methanol as diluent, in at least one reactor, and
(b) the reaction mixture leaving step (a) is subjected to a treatment to separate out the epichlorohydrin by extraction using an extraction solvent, in order to collect, on the one hand, an extract containing at least some of the extraction solvent, at least 10% of the epichlorohydrin produced, possibly an excess of allyl chloride and traces of methanol, and, on the other hand, a raffinate containing at least some of the diluent, at least some of the water, traces of epichlorohydrin and by-products,
(c) the extract is subjected to a distillation treatment in order to separate a first fraction containing the epichlorohydrin produced, possibly the excess allyl chloride and traces of methanol, from a second fraction containing the extraction solvent, which is recycled into the extraction step (b).

2. Process according to Claim 1, in which the first fraction obtained in the distillation step (c) is subjected to a distillation treatment (d) in order to remove any excess allyl chloride and the traces of methanol, which are recycled into the epoxidation step (a), and an epichlorohydrin-based product is collected and then dried in an azeotropic column.

3. Process according to Claim 1 or 2, in which the raffinate obtained in the extraction step (b) is subjected to a distillation (e) in order to separate a first fluid containing the water and by-products from a second fluid containing the methanol and traces of epichlorohydrin, which are recycled into the epoxidation step (a).

4. Process according to Claim 3, in which the first fluid is subjected to a purification treatment (f) in order to remove the by-products, and in which the purification treatment is performed by distillation in the presence of an organic compound or by extraction using an extraction liquid.

5. Process according to any one of Claims 1 to 4, in which the peroxide compound is hydrogen peroxide.

6. Process according to any one of Claims 1 to 5, in which the epichlorohydrin-based product contains at least 99.9% by weight of epichlorohydrin.

7. Process according to any one of Claims 1 to 6, in which the epichlorohydrin-based product contains chloro impurities in a total amount of less than or equal to 150 ppm by weight.

8. Process according to any one of Claims 1 to 7, in which the epichlorohydrin-based product contains methyl glycidyl ether in an amount of less than or equal to 250 ppm by weight.

9. Process according to any one of Claims 1 to 8, in which the epichlorohydrin-based product contains 2-methoxy-1-propanol in an amount of less than or equal to 100 ppm by weight.

10. Process according to any one of Claims 7 to 9, in which the total amount of chloro impurities is less than or equal to 100 ppm, the amount of methyl glycidyl ether is less than or equal to 200 ppm and the amount of 2-methoxy-1-propanol is less than or equal to 80 ppm.

## Patentansprüche

1. Verfahren zur Herstellung eines Produkts auf der Basis von Epichlorhydrin, gemäß dem
(a) man Allylchlorid umsetzt mit einer Peroxidverbindung in Gegenwart von Wasser, eines Katalysators und Methanol als Verdünnungsmittel in wenigstens einem Reaktor und
(b) man das aus der Stufe (a) kommende Reaktionsgemisch einer Behandlung zur Abtrennung des Epichlorhydrins durch Extraktion mit einem Extraktionslösungsmittel unterzieht und man einerseits einen Extrakt, der wenigstens einen Teil des Extraktionslösungsmittels, wenigstens 10% des erzeugten Epichlorhydrins, gegebenenfalls einen Allylchloridüberschuss und Methanolspuren enthält, und andererseits ein Raffinat gewinnt, das wenigstens einen Teil des Verdünnungsmittels, wenigstens einen Teil des Wassers, Epichlorhydrinspuren und Nebenprodukte enthält,
(c) man den Extrakt einer Destillationsbehandlung unterzieht, um eine erste Fraktion, die das erzeugte Epichlorhydrin, gegebenenfalls den Allylchloridüberschuss und Methanolspuren enthält, von einer zweiten Fraktion abzutrennen, die das Extraktionslösungsmittel enthält, das man in die Stufe zur Extraktion (b) zurückführt.

2. Verfahren gemäß Anspruch 1, worin man die erste, in der Stufe zur Destillation (c) erhaltene Fraktion einer Destillationsbehandlung (d) unterzieht, um den etwaigen Allylchloridüberschuss und die Methanolspuren zu entfernen, die man in die Stufe zur Epoxidierung (a) zurückführt, und man ein Produkt auf der Basis von Epichlorhydrin gewinnt, das anschließend in einer azeotropen Säule getrocknet wird.

3. Verfahren gemäß Anspruch 1 oder 2, worin man das in der Stufe zur Extraktion (b) erhaltene Raffinat einer Destillation (e) unterzieht, um ein erstes Medium, das das Wasser und Nebenprodukte enthält, von einem zweiten Medium abzutrennen, das das Methanol und Epichlorhydrinspuren enthält, die man in die Stufe zur Epoxidierung (a) zurückführt.

4. Verfahren gemäß Anspruch 3, worin man das erste Medium einer Behandlung zur Reinigung (f) unterzieht, um die Nebenprodukte zu entfernen, und worin die Behandlung zur Reinigung durch Destillation in Gegenwart einer organischen Verbindung oder durch Extraktion mit einer Extraktionsflüssigkeit durchgeführt wird.

5. Verfahren gemäß einem der Ansprüche 1 bis 4, worin die Peroxidverbindung Wasserstoffperoxid ist.

6. Verfahren gemäß einem der Ansprüche 1 bis 5, worin das Produkt auf der Basis von Epichlorhydrin wenigstens 99,9 Gew.-% Epichlorhydrin enthält.

7. Verfahren gemäß einem der Ansprüche 1 bis 6, worin das Produkt auf der Basis von Epichlorhydrin chlorhaltige Verunreinigungen in einer Gesamtmenge von kleiner oder gleich 150 Gew.-ppm enthält.

8. Verfahren gemäß einem der Ansprüche 1 bis 7, worin das Produkt auf der Basis von Epichlorhydrin Methylglycidylether in einer Menge von kleiner oder gleich 250 Gew.-ppm enthält.

9. Verfahren gemäß einem der Ansprüche 1 bis 8, worin das Produkt auf der Basis von Epichlorhydrin 2-Methoxypropan-1-ol in einer Menge von kleiner oder gleich 100 Gew.-ppm enthält.

10. Verfahren gemäß einem der Ansprüche 7 bis 9, worin die Gesamtmenge an chlorhaltigen Verunreinigungen kleiner oder gleich 100 ppm, die Menge an Methylglycidylether kleiner oder gleich 200 ppm und die Menge an 2-Methoxypropan-1-ol kleiner oder gleich 80 ppm ist.
